# DEMANDE DE BREVET EUROPEEN

(11) **EP 2 574 241 A1**
(43) Date de publication de la demande: **03.04.2013**
(21) Numéro de dépôt: 12306188.9
(22) Date de dépôt: 28.09.2012
(51) Int. Cl.: A23L 1/29, A23L 1/30, A23L 1/304, A23K 1/175, A23L 1/00, A61K 36/185, A61K 36/537, A61K 36/11, A61K 36/81

(54) **Specialite micronutritionnelle a base de carbonate de calcium et de vegetaux**

(30) Priorité: 30.09.2011 FR 1158846
(71) Demandeur: E ETHNO'PROSPECTIONS, 33170 Gradignan (FR)
(72) Inventeur: Hanras, Catherine, 33170 GRADIGNAN (FR)
(74) Mandataire: Cenatiempo, Julie Adeline Anne

(57) **Abrégé**

L'objet de l'invention est une composition obtenue à partir carbonate de calcium et de végétaux, et son utilisation comme complément alimentaire ou spécialité nutritionnelle utile dans la prévention et le traitement de certaines maladies, en particulier de maladies liées à des troubles du métabolisme du calcium.

## Description

La présente invention se rapporte à une composition particulière d'origine naturelle adaptée pour une utilisation comme complément alimentaire. L'invention vise également cette composition pour son application comme complément alimentaire, spécialité micronutritionnelle ou produit diététique, en particulier dans le cadre de la prévention ou du traitement des perturbations des fonctions métaboliques du calcium et des désordres physiopathologiques associés chez l'homme ou l'animal.

Le calcium, constituant organique sous sa forme ionisée Ca²⁺, est un micronutriment de régulation du métabolisme.

Malgré l'extrême prédominance du calcium osseux, la variable régulée n'est pas le contenu de l'organisme en calcium mais la concentration extracellulaire de calcium ionisé organique soluble (Ca²⁺) qui doit rester la plus constante possible. En effet, nos cellules sont soumises en permanence à une combinatoire de molécules qui régentent leur vie et leur mort en agissant à travers des systèmes de transduction déclenchant des cascades de réactions biochimiques intracellulaires. Or, le calcium Ca²⁺ joue un rôle universel dans ces mécanismes de transduction des signaux d'activation extra cellulaires, en tant que messager secondaire. Le contrôle des variations de l'homéostasie calcique constitue un dénominateur commun à de nombreuses étapes du développement et du fonctionnement normal ou pathologique de nos cellules et donc de notre organisme.

Parmi les nombreuses fonctions physiologiques extrêmement sensibles aux variations des concentrations plasmatiques en calcium sous forme ionisée organique Ca²⁺ ou calcémie, on peut citer l'excitabilité neuromusculaire et la contraction musculaire, la conduction et la transmission nerveuse, les mécanismes d'exocytose comme la libération des neurotransmetteurs, la coagulation sanguine, comme second messager de régulation intra et extra cellulaires pour la sécrétion d'hormones, la mobilité, la division et la cohésion cellulaire ou encore le maintien de l'intégrité des membranes et de manière indirecte la préservation et le maintien de l'équilibre physiologique du remodelage du tissu osseux et donc du capital osseux. En effet, lors de carences prolongées en minéraux, le calcium extra-osseux est maintenu constant par l'utilisation des réserves minérales de l'os au détriment de son équilibre physiologique.

Le calcium joue donc un rôle fondamental dans les voies métaboliques centrales responsables du bon fonctionnement de l'organisme et donc de sa santé. Les apports calciques moyens chez l'homme doivent permettre de :
- préserver la minéralisation osseuse, car l'homéostasie du calcium au niveau cellulaire est maintenue aux dépens des réserves échangeables des os, et
- compenser les pertes endogènes irréductibles, de l'ordre de 350 mg/j chez l'adulte (perte urinaire journalière, excrétion calcique, sueur, etc.).

La calcium n'est pas apporté à l'organisme sous forme ionisée. C'est la présence d'acide chlorhydrique (pH 2) dans le suc gastrique au niveau de l'estomac qui permet la formation du calcium sous forme ionisé organique Ca²⁺ soluble et de chlorure de calcium soluble. Le Ca²⁺ passe ensuite dans l'intestin grêle où il devient insoluble (pH ≥ 6). Or, c'est seulement sous sa forme soluble que le calcium Ca²⁺ peut être absorbé au niveau des parois intestinales à la fois par un transport actif transcellulaire et un transport passif paracellulaire, dépendant de nombreux paramètres. Le coefficient net d'absorption intestinale, c'est-à-dire la fraction réellement retenue par l'organisme après digestion et absorption d'un nutriment ne dépasse pas pour le calcium sous sa forme organique ionisée 25 à 30%.

C'est pourquoi, les compositions et formulations à base de carbonate de calcium existants actuellement (par exemple carbonate de calcium + vitamine D) sont peu efficaces, car lorsqu'elles sont ingérées, le pH > 6,0 rend insoluble la majeur partie des ions qui ne peuvent pas être absorbés.

Si la stabilité de la calcémie est dépendante de l'absorption intestinale de Ca²⁺, elle est également influencée par la réabsorption calcique rénale et la résorption osseuse. Ces mécanismes sont contrôlées par les mécanismes de régulation homéostasique eux-mêmes dépendants de l'apport en certains minéraux comme le magnésium Mg²⁺ et/ou le zinc Zn²⁺.

Le magnésium est un cofacteur de l'assimilation du Ca²⁺, de la minéralisation osseuse et de la balance électrolytique. Il joue un rôle synergique avec le Ca²⁺ au niveau des voies métaboliques en activant plus de 300 enzymes des métabolismes énergétiques (notamment les kinases).

L'ion zinc, cofacteur de plus de 200 métalloenzymes, joue également un rôle synergique avec le Ca²⁺ au niveau des voies métaboliques. Il est nécessaire notamment à la synthèse de l'insuline, des immunoglobulines et des acides nucléiques, et favorise la multiplication cellulaire et la cicatrisation. Il intervient aussi dans le métabolisme de la vitamine A et des acides gras, dans la protection des cellules contre le stress oxydatif, etc.

L'apport en micronutriments minéraux sous forme assimilable est donc d'une extrême importance pour l'organisme.

Toutefois, un simple apport en ces micronutriments essentiels n'est pas suffisant car leur efficacité dépend de leur absorption ou assimilation (quantité qui passe la barrière ou muqueuse intestinale) et de leur biodisponibilité (quantité qui est disponible et utilisable par la cellule) qui sont fonction de paramètres multifactoriels comme :
- la source alimentaire en particulier la nature et la forme du minéral ainsi que la balance chimique ;
- la composition du bol alimentaire, car la présence d'autres nutriments ou substances capables d'influencer leurs mécanismes d'absorption peut créer des interactions complexes ;
- la fenêtre et le mode d'absorption :
   ○ transport actif transcellulaire au niveau du duodénum et jéjunum supérieur, processus saturable nécessitant de l'énergie sous forme ATP par les canaux ioniques et transporteurs, dépendant de la vitamine D, et
   ○ transport passif paracellulaire, majoritairement au niveau de l'iléon, qui correspond à une diffusion passive au niveau des jonctions intercellulaires, dont les jonctions serrées, selon le gradient électrochimique ;
- l'homéostasie des statuts en micronutriments essentiels ;
- l'état physiologique des muqueuses gastrique et intestinale.

C'est pourquoi l'objectif de la présente invention est de proposer une composition d'origine naturelle ayant des fonctionnalités qui permettent l'optimisation de l'assimilation et de la biodisponibilité des apports en micronutriments essentiels, en particulier de calcium sous forme ionisée active Ca²⁺, en vue notamment de prévenir et/ou lutter contre les dysfonctionnements et pathologies liées aux troubles des métabolismes du calcium chez l'homme ou l'animal.

Pour répondre à son objectif, l'invention vise une composition adaptée pour une utilisation comme complément alimentaire ou spécialité micronutritionnelle pour l'homme ou l'animal, comprenant au moins du calcium et constituée par mélange d'au moins :
- une matière première comprenant au moins un carbonate de calcium,
- un broyat d'au moins une partie d'au moins une plante de la famille des *Verbenaceae,* et
- un broyat d'au moins une partie d'au moins une plante du genre *Equisetum.*

Avantageusement, une telle composition est constituée d'éléments minéraux et végétaux qui permettent l'optimisation de l'assimilation et de la biodisponibilité des apports en micronutriments essentiels en agissant de façon synergique au sein de l'organisme. Leur action porte en particulier :
- sur la sphère ostéoarticulaire,
- sur les métabolismes énergétique, lipidique et protéinique, et
- sur les défenses immunitaires.

Les végétaux particuliers utilisés permettent de protéger et solubiliser les minéraux, notamment les cations organiques calcium Ca²⁺ dans l'intestin grêle. Aussi, malgré le pH > 6,0 dans l'intestin, le Ca²⁺ reste soluble et/ou dans un état chimique qui lui permet d'être absorbé par voie paracellulaire et/ou transcellulaire.

Selon un autre avantage, les végétaux particuliers utilisés permettent également d'augmenter la perméabilité des jonctions interentérocytaire stimulant la diffusion passive au niveau de l'intestin selon le gradient de concentration électrochimique.

D'autres caractéristiques et avantages ressortiront de la description en détail qui va suivre de l'invention.

Selon un premier aspect, l'invention se rapporte donc à une composition adaptée pour une utilisation comme complément alimentaire ou spécialité micronutritionnelle pour l'homme ou l'animal, comprenant au moins du calcium et constituée par mélange d'au moins :
- une matière première, préférentiellement sous forme de broyat, comprenant au moins un carbonate de calcium,
- un broyat d'au moins une partie d'au moins une plante de la famille des *Verbenaceae,* et
- un broyat d'au moins une partie d'au moins une plante du genre *Equisetum.*

Préférentiellement, la composition selon l'invention comprend également de la silice organique.

Selon une variante particulièrement adaptée, la composition comprend également du magnésium, et la matière première comprenant au moins un carbonate de calcium est un broyat d'au moins une roche sédimentaire carbonatée de carbonate double de calcium et de magnésium.

Par « complément alimentaire » au sens de l'invention, on entend un complément alimentaire tel que défini par la Directive européenne 2002/46/CE article 2, à savoir « des denrées alimentaires dont le but est de compléter le régime alimentaire normal et qui constituent une source concentrée de nutriments ou d'autres substances ayant un effet nutritionnel ou physiologique, seuls ou combinés, commercialisés sous forme de dose, à savoir les formes de présentation telles que les gélules, les pastilles, les comprimés, les pilules et autres formes similaires ainsi que les sachets de poudre, les ampoules de liquide, les flacons munis d'un compte-goutte et les autres formes analogues de préparations liquides ou en poudres destinées à être prises en unités mesurées de faible quantité ».

Par « spécialité micronutritionnelle » au sens de l'invention on entend un complément alimentaire dont la composition contient des micronutriments essentiels.

Par « broyat » au sens de l'invention on entend tout produit obtenu par broyage, une poudre quelle que soit la granulométrie (morceaux ou granulométrie très fine).

Par « au moins une partie d'une plante » au sens de l'invention on entend une partie de la plante ou toute la plante. Cela inclue le totum de plante (la plante entière comprenant à la fois les parties au-dessus du sol et dans le sol), le totum aérien (toute partie de la plante se situant au-dessus du sol) et les feuilles entières. Préférentiellement il s'agit du totum aérien ou de feuilles entières de la plante.

La composition selon l'invention est donc constituée par au moins un broyat comprenant au moins un carbonate de calcium. Il peut s'agir par exemple de broyat de roche contenant du carbonate de calcium, de broyat de coquille d'huître ou de coquille d'oeuf, etc.

Préférentiellement, la matière première comprenant au moins un carbonate de calcium est un broyat d'au moins une roche sédimentaire de carbonate double de calcium et de magnésium (calcomagnésien).

Par roche sédimentaire carbonate double de calcium et de magnésium au sens de l'invention, on entend les roches sédimentaires contenant du calcium et du magnésium. Préférentiellement il s'agit de roches sédimentaires contenant du calcium et du magnésium dans une même structure cristalline.

De façon préférée, les roches sédimentaires carbonate double de calcium et de magnésium sont des roches sédimentaires carbonatées caractérisées par une composition minéralogique comprenant de la dolomite. La dolomite est une espèce minérale formée de carbonate double de calcium et de magnésium de formule chimique Ca,Mg(CO₃)₂ avec des traces de Fe, Mn, Pb, Zn, Rb, Sr.

Il peut s'agir de roches carbonatées constituées principalement de dolomite ou encore de roches dites intermédiaires ou calcaires dolomitiques ou de calcaires magnésiens dont la composition minéralogique est constituée notamment de cristaux rhomboédriques de calcite (CaCO₃), de dolomite (Ca,Mg(CO₃)₂) et/ou de quartz (SiO₂). Ces roches peuvent être constituées par d'autres composants mineurs.

Un exemple de roche pouvant être utilisée selon l'invention est une dolomie composée de 90 à 98% de dolomite.

Le broyat de matière première comprenant au moins un carbonate de calcium est obtenue par broyage de ladite matière première. Lorsqu'il s'agit d'une roche, le broyat est préférentiellement obtenu par broyage de la roche sédimentaire dans son ensemble sans aucune extraction préalable.

Selon une variante, la composition selon l'invention est constituée par au moins deux broyats de roches sédimentaires différentes.

Selon un mode de réalisation particulièrement adapté, une des deux roches contient de la silice et l'autre n'en contient pas, de sorte qu'elle est entièrement cristalline de préférence sans zone amorphe.

La roche sans silice peut par exemple être choisie parmi les calcaires et les craies, et la roche contenant de la silice peut par exemple être choisie parmi les dolomies ou le loess.

Un couple de roches sédimentaires préféré est craie et dolomie. Préférentiellement la matière première comprenant au moins un carbonate de calcium est broyée avec une granulométrie inférieure à 20µm, de façon à favoriser l'absorption intestinale des micronutriments.

La composition selon l'invention est également constituée d'au moins deux broyats de végétaux, à savoir ;
- un broyat d'au moins une partie d'au moins une plante de la famille des *Verbenaceae,* et
- un broyat d'au moins une partie d'au moins une plante du genre *Equisetum.*

Le broyat d'au moins une partie d'une plante au sens de l'invention est la partie de la plante, préférentiellement séchées, qui a été broyée. Très préférentiellement aucune extraction n'a été réalisée et tous les constituants de la partie de la plante utilisée sont conservés. De façon préférée, les plantes sont plantées, cultivées, récoltées et séchées de façon à conserver toute leur activité ou potentiel d'efficacité observé lors des usages traditionnels, puis broyées, préférentiellement finement.

Selon un mode de réalisation particulièrement adapté, les plantes sont broyées de façon à obtenir une granulométrie inférieure à 5 µm, encore plus préférentiellement inférieure à 1 µm. Ceci permet d'obtenir une grande surface de contact ou surface spécifique entre la muqueuse gastro-intestinale et les micronutriments essentiels et améliore l'efficacité.

De façon préférée, pour le broyat d'au moins une partie d'au moins une plante de la famille des *Verbenaceae* de la composition selon l'invention, ladite plante de la famille des *Verbenacea* est choisie parmi :
- *Aloysia citriodora,*
- *Lippia alba,*
- *Aloysia polystachya,*
- *Aloysia gratissima,*
- *Verbena officinalis*

Préférentiellement, le broyat d'au moins une partie d'une plante de la famille des *Verbenaceae* de la composition selon l'invention, comprend au moins un broyat d'au moins une partie, (préférentiellement totum aérien ou feuilles entières) *d'Aloysia citriodora.*

Par ailleurs, le broyat d'au moins une partie d'une plante du genre *Equisetum* de la composition selon l'invention, est préférentiellement constituée d'un broyat d'au moins une partie, (préférentiellement totum aérien ou feuilles entières) d'une plante d'*Equisefum arvense L.* et/ou d'*Equisetum giganteum L.*

Le mélange de broyat de la composition selon l'invention peut également contenir :
- un minéral ou une roche contenant du zinc, et/ou
- un broyat d'au moins une partie de *Solanum glaucophyllum,* et/ou
- un broyat d'au moins une partie d'une plante du genre *Stevia,* et/ou
- un broyat d'au moins une partie d'une plante de la famille des *Lamiaceae,* en particulier du genre *Salvia.*

Le minéral ou la roche contenant du zinc, permet d'enrichir encore la composition en Zn²⁺. Il s'agit préférentiellement d'oxyde de zinc.

La présence de *Solanum glaucophyllum* permet un apport en vitamine D sous forme de glycosides de 1,25-dihydroxyvitamine D. Les feuilles sont préférentiellement prélevées avant floraison et séchées. L'apport de vitamine D permet entre autre de favoriser le transport actif transcellulaire des minéraux au niveau de l'intestin et d'améliorer l'absorption calcique intestinale.

La plante du genre *Stevia* joue un rôle d'édulcorant naturel. Il peut s'agir notamment de *Stevia rebaudiana.*

Préférentiellement, dans la composition selon l'invention les constituants minéraux (la ou les matière(s) première(s) contenant du carbonate de calcium, préférentiellement la ou les broyat(s) de roche(s) sédimentaire(s), et éventuellement le ou les minéral(aux) ou roche(s) contenant du zinc) représentent 65 à 85% en poids de l'extrait sec de la composition selon l'invention, et les constituants végétaux (broyat(s) de végétaux) représentent entre 15 et 35% en poids de l'extrait sec de la composition.

Selon un mode de réalisation préférée, la composition est constituée exclusivement par le mélange :
- d'un broyat d'une roche sédimentaire carbonatée de carbonate double de calcium et de magnésium contenant de la silice,
- d'un broyat d'une roche sédimentaire carbonatée de carbonate double de calcium et de magnésium sans silice,
- d'un minéral contenant du zinc,
- d'un broyat d'au moins une partie d'au moins une plante de la famille des *Verbenaceae,*
- d'un broyat d'au moins une partie d'au moins une plante du genre *Equisetum,*
- d'un broyat d'au moins une partie de *Salanum glaucophyllum,* et
- d'un broyat d'au moins une partie d'une plante du genre *Stevia,*
- d'un broyat d'au moins une partie d'une plante de la famille des *Lamiaceae,* en particulier du genre *Salvia.*

Préférentiellement, la composition est constituée par le mélange de :
- entre 10 et 40% d'un broyat d'une roche sédimentaire carbonatée de carbonate double de calcium et de magnésium contenant de la silice,
- entre 20 et 45% d'un broyat d'une roche sédimentaire carbonatée de carbonate double de calcium et de magnésium sans silice,
- entre 5 et 15% d'un minéral ou d'une roche contenant du zinc,
- entre 10 et 40% d'un broyat d'au moins une partie d'une plante de la famille des *Verbenaceae,*
- entre 5 et 20% d'un broyat d'au moins une partie d'une plante du genre *Equisetum,*
- entre 1 et 10% d'un broyat d'au moins une partie de *Solanum glaucophyllum,* et
- entre 1 et 5% d'un broyat d'au moins une partie d'une plante du genre *Stevia,*
- entre 10 et 25% d'un broyat d'au moins une partie d'une plante de la famille des *Lamiaceae,* en particulier du genre *Salvia,*
les pourcentages étant exprimés en poids de matière sèche de la composition.

Préférentiellement le broyat d'au moins une partie des plantes est un broyat du totum aérien ou de feuilles entières de la plante.

Selon un autre aspect, la composition peut être caractérisée par les composés qu'elle contient. La composition selon l'invention comprend les éléments contenus dans les différents constituants de la composition (broyat de matière première contenant au moins un carbonate de calcium, broyats de feuilles entières de végétaux, etc.). Elle comprend au moins du calcium. Très préférentiellement, elle contient également de la silice organique ou silice biogène.

Le calcium provient de la matière première comprenant au moins un carbonate de calcium.

La silice organique est sous forme de silicium organique ou silice biogène ou de silicates hydrosolubles, provenant des végétaux.

Préférentiellement, la composition contient également du magnésium, en particulier lorsque la matière première contenant au moins un carbonate de calcium est un broyat de roche sédimentaire carbonatée de carbonate double de calcium et de magnésium. Dans ce cas, le calcium et le magnésium proviennent de la ou des roche(s) sédimentaire(s). Préférentiellement, ils proviennent de la même structure cristalline.

De façon préférée, la composition selon l'invention comprend également les micronutriments suivants :
- zinc, et/ou
- carbonate de calcium CaCO₃, et/ou
- phosphore P₂O₃, et/ou
- phytohormones, en particulier les isoflavones et des lignanes, et/ou
- des complexes biomoléculaires, agents facilitateurs (« enhancers »), et/ou
- des antioxydants, en particulier des flavonoïdes et des polyphénols, et/ou
- des petites molécules organiques, notamment des acides nucléiques, des acides aminés, des peptides, des ions, et/ou
- des macromolécules, en particulier des polypeptides et des glucides complexes.

Très préférentiellement, la composition selon l'invention comprend l'ensemble de ces éléments.

Comme le zinc, le carbonate de calcium et le phosphore sont d'origine minérale. Les autres éléments sont d'origine végétale.

La composition selon l'invention se présente préférentiellement sous forme de broyat conditionnable en gélules, sachets ou pots, ou comme additifs pour des préparations diététiques.

Préférentiellement, elle présente une densité D¹⁰ et D₅₀₀ comprise entre 0,5 et 0,9 (densité tapée mesurée avec un voluménomètre à tassement) et une teneur en eau comprise entre 3,5 et 4,5% (mesure de la perte à dessiccation). La composition selon l'invention peut être obtenue selon un procédé comprenant les étapes suivantes :
- récolte et séchage préférentiellement selon des procédés dérivés des savoir-faire traditionnels,
- broyage individuel de chacune des plantes, avec une granulométrie inférieure à 5µm, préférentiellement inférieure à 1 µm, par exemple à l'aide d'un broyeur à hélice(s) ou boules,
- stockage individuel des plantes broyées dans des containers opaques,
- broyage individuel de deux matières premières comprenant au moins un carbonate de calcium, préférentiellement des roches, avec un granulométrie inférieure à 20µm,
- mélange d'une des matières premières broyées comprenant du carbonate de calcium, avec de l'oxyde de zinc,
- ajout de la seconde matière première broyée comprenant du carbonate de calcium et mélange,
- ajout des deux plantes broyées et mélange.

Avantageusement, chacun des éléments présents dans la composition selon l'invention agit en synergie avec les autres pour optimiser l'assimilation et la biodisponibilité des apports en minéraux au sein de l'organisme. L'assemblage proportionné et synergique des différents constituants minéraux et végétaux apporte à la fois :
- les micronutriments essentiels, en particulier calcium, magnésium et zinc :
   ○ pour la constitution, la régulation, le renforcement et le maintien de la santé de la sphère ostéoarticulaire de façon optimale et durable,
   ○ pour corriger les désordres physiopathologiques dus à des perturbations des métabolismes du calcium, du magnésium et/ou du zinc.
- la densité nutritionnelle intégrative ou biofonctionnalité : c'est-à-dire des complexes moléculaires et/ou macromoléculaires et/ou phytomicronutriments divers d'origine végétale qui permettent une assimilation et une biodisponibilité optimisée desdits micronutriments essentiels ainsi que l'amélioration des déficits potentiels des métabolismes et mécanismes cellulaires associés.

Les éléments issus des constituants minéraux (la matière première contenant du carbonate de calcium, préférentiellement la ou les broyat(s) de roche(s) sédimentaire(s), et éventuellement le minéral ou la roche contenant du zinc) de la composition selon l'invention apportent les minéraux et oligoéléments essentiels. Avantageusement, lorsque les constituants minéraux sont des roches sédimentaires carbonatées de carbonate double de calcium et de magnésium, le calcium et le magnésium proviennent de la même structure cristalline et cette forme d'administration est très importante en terme d'efficacité de la composition.

Ces minéraux et oligoéléments jouent un rôle notamment :
- au niveau du capital osseux à la fois pour la croissance, la constitution de l'os et pour l'activité de remodelage (résorption et formation/minéralisation de la matrice organique)
- au niveau des mécanismes régulateurs homéostasiques pour la régulation de la calcémie,
- au niveau du métabolisme du calcium extra-osseux,
- au niveau de l'équilibre acido-basique de façon à pouvoir neutraliser l'acidité gastrique et présenter un effet antiacide,
- au niveau des modulateurs enzymatiques et régulateurs allostériques,
- au niveau des défenses immunitaires, et
- au niveau du métabolisme énergétique.

Par ailleurs, les éléments issus des constituants végétaux de la composition selon l'invention jouent également un rôle important.

La silice organique stimule la synthèse de collagène dans les tissus conjonctifs osseux, cartilagineux et tendineux. Elle est également impliquée dans le renouvellement des tissus conjonctifs ainsi que dans la fixation du calcium dans le tissu osseux, et stabilise et protège les membranes cellulaires de la péroxydation cytotoxique.

Les phytohormones quant à elles peuvent atténuer et soulager les troubles attribués à la ménopause.

Les autres éléments issus des constituants végétaux jouent un rôle d'agents de densité nutritionnelle car ils influencent les mécanismes d'absorption ou d'assimilation des autres éléments (minéraux, oligoéléments, silice organique et phytohormones).

En particulier, les constituants végétaux permettent de protéger et solubiliser les minéraux, notamment les cations organiques calcium Ca²⁺ dans l'intestin grêle. Ils permettent également d'augmenter la perméabilité des jonctions interentérocytaire stimulant la diffusion passive au niveau de l'intestin selon le gradient de concentration électrochimique.

Avantageusement, la présence des différents composants d'origine naturelle agissant en synergie, permet à la composition d'agir en particulier sur la sphère ostéoarticulaire, sur les métabolismes énergétique, lipidique et protéinique, et/ou sur les défenses immunitaires.

La composition selon l'invention est donc particulièrement adaptée pour son application comme complément alimentaire ou spécialité micronutritionnelle à visée humaine ou animale, en particulier dans la prévention et/ou le traitement des troubles du métabolisme de certains minéraux et oligoélément, préférentiellement dans la prévention et/ou le traitement des troubles du métabolisme du calcium, en renforçant les statuts physiologiques des métabolismes du calcium.

La composition selon l'invention peut notamment être utile comme complément alimentaire ou spécialité micronutritionnelle dans la prévention et le traitement :
- des dysfonctionnements et pathologies liées aux troubles des métabolismes du calcium chez l'homme ou l'animal,
- des maux de la sphère ostéo-articulaire et leurs effets connexes chez l'homme ou l'animal,
- de l'ostéoporose, de l'arthrose, des troubles de la croissance ou des désordres physiopathologiques dus à l'anorexie chez l'homme,
- pour renforcer les défenses immunitaires, de l'homme ou de l'animal,
- en compléments de soins des effets secondaires dus à certains traitement engendrant des perturbations des statuts calciques.

La composition selon l'invention peut ainsi contribuer à réduire les risques de survenue de certaines pathologies chroniques dues aux formes de malnutrition actuelles et aux processus de vieillissement de l'organisme : déséquilibres et/ou carences nutritionnels et baisse de biodisponibilité des micronutriments essentiels.

En maintenant l'homéostasie calcique, la composition selon l'invention peut aussi contribuer à la prévention :
- des pathologies de surpoids et syndrome métabolique,
- des cancers hormonaux-dépendants (colons, seins),
- des maladies cardiovasculaires.

Elle peut également être utile comme complément alimentaire ou spécialité micronutritionnelle pour aider à mieux surmonter les effets secondaires d'une chimiothérapie ou d'une radiothérapie chez l'homme ou encore de traitements à base de cortisone, d'une déficience ou carence en vitamine D.

Chez l'homme, elle peut aussi être utilisée comme ou dans des préparations diététiques ou en lithothérapie déchélatrice.

Enfin, la composition selon l'invention peut aussi être utilisée chez l'animal, comme additif et complément alimentaire à usage vétérinaire. Elle peut alors se présenter sous forme de broyat seul ou mélangée à des préparations ou formulations de type granulés ou granulats, pierre à sel, etc. Chez l'animal, elle est particulièrement utile pour :
- augmenter les apports calciques, en particulier pour la constitution du capital osseux, développement et renforcement du système immunitaire (immunité non spécifique),
- lutter contre les déficiences ou carences en vitamine D, afin notamment d'optimiser et/ou d'augmenter les mécanismes de métabolisation (par exemple dans le cas des ruminants qui fabriquent des protéines à partir de fermentations de fourrage ou de granulés),
- jouer un rôle de tampon naturel pH ≥5, de façon à prévenir et/ou tamponner l'acidose métabolique et les déséquilibres acido-basiques dans le cas d'élevage intensif de ruminants provoqués par une alimentation quantitative pour métabolisation intensive.

La composition selon l'invention doit en outre être utilisée à des faibles doses. Il doit s'agir d'une homéonutrition pour une meilleure biodisponibilité et régulation homéostasique du calcium extracellulaire. En effet, si l'on consomme trop de calcium par des apports journaliers élevés pendant trop longtemps, l'organisme peut perdre sa capacité à contrôler les mécanismes de régulation homéostasiques.

De plus, l'efficacité de la composition selon l'invention en terme d'assimilation et de biodisponibilité des micronutriments est encore augmentée par un fractionnement de la dose au cours de la journée et par une administration en cures.

L'invention est à présent illustrée par des exemples illustratifs, non limitatifs.

### Exemples de compositions

### Exemple 1 : composition B1

La composition B1 est constituée des éléments suivants (en pourcentage de poids total de matière sèche de la composition) :
- 16% de roche sédimentaire CaMg(CO₃)₂ + SiOz [ingrédient 1]
- 45% de roche sédimentaire CaMg(CO3)₂ [ingrédient 2]
- 4% de minéral contenant du zinc [ingrédient 3]
- 14% de *Aloysia citriodora* [ingrédient 4]
- 7% de sauge (genre *Salvia*) [ingrédient 5]
- 11*%* d'*Equisetum arvense L.* [ingrédient 6]
- 2% de *Solanum glaucaphyllum* [ingrédient 7]
- 1% de *Stevia rebaudiana* [ingrédient 8].

Cette composition peut être obtenue par la mise en oeuvre du procédé suivant :
- Mélange minéral : mélange de l'ingrédient 1 avec l'ingrédient 3, puis ajout de l'ingrédient 2 et mélange,
- Mélange des végétaux : mélange des ingrédients 4, 7 et 8 et ajout en mélange des ingrédients 5 et 6,
- Le mélange des extraits végétaux est ajouté au mélange minéral, puis l'ensemble est mélangé pour obtenir le produit final.

### Exemple 2 : composition B2

La composition B2 est constituée des éléments suivants (en pourcentage de poids total de matière sèche de la composition) :
- 30% de roche sédimentaire CaMg(CO3)2 + SiO2 [ingrédient 1]
- 45% de roche sédimentaire CaMg(CO3)2 [ingrédient 2]
- 10% de Verbena officinalis [ingrédient 3]
- 9% de sauge (genre Salvia) [ingrédient 4]
- 6% d'Equisetum giganteum L. [ingrédient 5]

Cette composition peut être obtenu par la mise en oeuvre du procédé suivant :
- Mélange minéral : mélange de l'ingrédient 1 avec l'ingrédient 2,
- Mélange des végétaux : mélange des ingrédients 3, 4 et 5,
- Le mélange des extraits végétaux est ajouté au mélange minéral, puis l'ensemble est mélangé pour obtenir le produit final.

### Exemples d'applications

La composition selon l'invention peut se présenter sous forme de gélule contenant 500mg de poudre totale, destinée à une administration en cures.

### Exemple 3 : application chez l'enfant de plus de 8 ans et l'adolescent

La composition selon l'invention peut être utilisée chez l'enfant de plus de 8 ans et l'adolescent pour :
○ une aide à la croissance : élaboration du squelette et donc acquisition du capital osseux,
○ la prévention des risques ostéoarticulaires,
○ le développement du système immunitaire,
○ permettre de diminuer le risque de fracture de fatigue des jeunes sportifs ayant un entrainement intensif,

La posologie conseillée est la suivante :
- cures de 2 mois avec un mois d'arrêt entre chaque cure,
- 1 gélule par jour de préférence au dîner à raison de 3 jours par semaine.

### Exemple 4 : application chez l'adulte en pré ou post-ménopause

La composition selon l'invention peut être utilisée chez l'adulte en pré ou post-ménopause pour :
○ une aide au maintien et/ou rétablissement du capital « ostéo-articulaire » et ses métabolismes associés,
○ une diminution des risques d'ostéoporose,
○ un renforcement des défenses naturelles.

La posologie conseillée est la suivante :
- cures de 2 mois avec un mois d'arrêt entre chaque cure,
- 2 gélules par jour de préférence 1 gélule au petit déjeuner et une gélule au dîner à raison de 5 jours par semaine.

### Exemple 5 : application chez le sportif

La composition selon l'invention peut être utilisée chez le sportif pour aider à la préparation et à la récupération lors d'exercices soutenus et/ou intenses, du capital calcium extracellulaire.

La posologie est conseillée par les entraîneurs en fonction de l'activité sportive et du niveau.

### Exemple 6 : application chez l'adulte de plus de 70 ans

L'efficacité de l'absorption intestinale du calcium s'atténue avec l'âge, associée la plupart du temps à une diminution des apports nutritionnel et à une déficience du statut en vitamine D.

La composition selon l'invention peut donc être utilisée chez l'adulte de plus de 70 ans pour :
○ une aide au maintien du capital « ostéo-articulaire »,
○ une aide à la régulation des métabolismes associés,
○ une stimulation des défenses naturelles,
○ une aide à une bonne conduction de l'influx nerveux et motricité.

La posologie conseillée est la suivante :
- Traitement de fond :
   ○ cure de 3 mois selon le protocole suivant :
      ■ pendant 3 semaines : 4 gélules par jour, 2 au petit déjeuner et 2 de préférence au dîner à raison de 5 jours par semaine,
      ■ pendant 10 semaines : 2 gélules par jour, de préférence au dîner à raison de 3 jours par semaine.
   ○ arrêt de 2 à 3 semaines en fonction des effets constatés,
   ○ cure de 2 mois selon le protocole suivant : 2 gélules par jour, de préférence 1 au petit déjeuner et 1 au dîner à raison de 3 ou 5 jours par semaine.
- Traitement intersaisons (2mois) : 2 gélules par jour de préférence au dîner à raison de 3 jours par semaine.

## Revendications

1. Composition adaptée pour une utilisation comme complément alimentaire ou spécialité micronutritionnelle pour l'homme ou l'animal, comprenant au moins du calcium et constituée par mélange d'au moins :
- une matière première comprenant au moins un carbonate de calcium,
- un broyat d'au moins une partie d'au moins une plante de la famille des *Verbenaceae,* et
- un broyat d'au moins une partie d'au moins une plante du genre *Equisetum.*

2. Composition selon la revendication 1, **caractérisée en ce qu'**elle comprend également du magnésium, et **en ce que** la matière première comprenant au moins un carbonate de calcium est un broyat de roche sédimentaire carbonatée de carbonate double de calcium et de magnésium.

3. Composition selon la revendication 1 ou 2, **caractérisée en ce qu'**elle comprend également les micronutriments suivants : silice organique, zinc, phosphore et/ou phytohormones.

4. Composition selon l'une des précédentes revendications, **caractérisée en ce qu'**elle est également constituée de broyat d'au moins une partie de *Solanum glaucophyllum.*

5. Composition selon l'une quelconque des précédentes revendications, **caractérisée en ce qu'**elle est également constituée de broyat d'au moins une partie d'une plante du genre *Stevia.*

6. Composition selon l'une des revendications 2 à 5, **caractérisée en ce qu'**elle est également constituée de broyat d'une autre roche sédimentaire carbonatée de carbonate double de calcium et de magnésium.

7. Composition selon la revendication 6, **caractérisée en ce qu'**une des deux roches comprend de la silice et l'autre roche n'en contient pas.

8. Composition selon l'une quelconque des précédentes revendications, **caractérisée en ce qu'**elle est également constituée d'un minéral ou d'une roche contenant du zinc.

9. Composition selon la revendication 8, **caractérisée en ce que** l'élément minéral contenant du zinc est l'oxyde de zinc.

10. Composition selon l'une quelconque des précédentes revendications, **caractérisée en ce que** ladite plante de la famille des *Verbenaceae* est choisie parmi :
- *Aloysia citriodora,*
- *Lippia alba,*
- *Aloysia polystachya,*
- *Aloysia gratissima,* et
- *Verbena officinalis.*

11. Composition selon l'une quelconque des précédentes revendications, **caractérisée en ce que** la plante de la famille des *Equisetaceae* est *Equisetum arvense L* ou *Equisetum giganteum L.*

12. Composition selon l'une quelconque des revendications 2 à 11, **caractérisée en ce que** la ou les matière(s) première(s) comprenant du carbonate de calcium éventuellement additionnée(s) d'une ou plusieurs roche(s) ou minéral(aux) contenant du zinc représente(nt) entre 65 et 85% en poids de l'extrait sec de la composition et les broyats de végétaux représentent entre 15 et 35% en poids de l'extrait sec de la composition.

13. Composition selon l'une quelconque des précédentes revendications, **caractérisée en ce qu'**elle se présente sous forme de broyat conditionnable en gélules, sachets ou pots.

14. Composition selon l'une quelconque des précédentes revendications pour son application comme complément alimentaire ou spécialité micronutritionnelle à visée humaine ou animale.

15. Composition selon l'une quelconque des précédentes revendications pour son application comme complément alimentaire ou spécialité micronutritionnelle dans la prévention et/ou le traitement des dysfonctionnements et pathologies liées aux troubles des métabolismes du calcium chez l'homme ou l'animal.

16. Composition selon l'une quelconque des précédentes revendications pour son application comme complément alimentaire ou spécialité micronutritionnelle dans la prévention et/ou le traitement des maux de la sphères ostéo-articulaire et leurs effets connexes chez l'homme ou l'animale.

17. Composition selon l'une quelconque des précédentes revendications pour son application comme complément alimentaire ou spécialité micronutritionnelle dans la prévention et/ou le traitement de l'ostéoporose, de l'arthrose, des troubles de la croissance ou des désordres physiopathologiques dus à l'anorexie chez l'homme.

18. Composition selon l'une des revendications 1 à 14 pour son application comme complément alimentaire ou spécialité micronutritionnelle pour aider à mieux surmonter les effets secondaires d'une chimiothérapie ou d'une radiothérapie chez l'homme, ou de traitements à base de cortisone ou de traitements engendrant des perturbations des statuts calciques chez l'homme ou l'animal.

19. Composition selon l'une des revendications 1 à 14 pour son application comme complément alimentaire ou spécialité micronutritionnelle pour renforcer les défenses immunitaires de l'homme ou de l'animal.

20. Composition selon l'une des revendications 1 à 14 pour son application comme complément alimentaire ou spécialité micronutritionnelle pour la prévention :
- des pathologies de surpoids et syndrome métabolique,
- des cancers hormonaux-dépendants,
- des maladies cardiovasculaires.

21. Composition selon l'une des revendications 1 à 14 pour son application comme ou dans des préparations diététiques.
